# EUROPÄISCHE PATENTSCHRIFT

(11) **EP 1 262 146 B1**
(45) Veröffentlichungstag und Bekanntmachung des Hinweises auf die Patenterteilung: **11.08.2004**
(21) Anmeldenummer: 02011886.5
(22) Anmeldetag: 29.05.2002
(51) Int. Cl.: A61B 6/03, A61B 19/08

(54) **Abdeckung für eine tunnelförmige medizinische Einrichtung sowie medizinische Einrichtung**
Tunnellike medical device and cover for it
Appareil médical en forme de tunnel et son élément protecteur

(30) Priorität: 31.05.2001 DE 10126465
(43) Veröffentlichungstag der Anmeldung: 04.12.2002
(73) Patentinhaber: Paul Hartmann AG, 89504 Heidenheim (DE)
(72) Erfinder: Braun, Anette, 89518 Heidenheim (DE)
(74) Vertreter: Dreiss, Fuhlendorf, Steimle & Becker

(56) Entgegenhaltungen:
- US-A- 4 910 819
- PATENT ABSTRACTS OF JAPAN vol. 1995, no. 09, 31. Oktober 1995 (1995-10-31) & JP 07 148147 A (TOSHIBA CORP), 13. Juni 1995 (1995-06-13)

## Beschreibung

Die Erfindung betrifft eine Abdeckung für eine tunnelförmige medizinische Einrichtung, insbesondere einen Computer- oder Kernspintomographen.

So weist beispielsweise ein Computertomograph einen Strahlensender und einen Strahlenempfänger auf, die eine tunnelförmige Öffnung zur Aufnahme eines Patienten oder Untersuchungsobjektes umgeben.

Bei einem Magnetresonanzgerät oder Kernspintomographen ist eine Aufnahmeeinrichtung mit einer Magneteinrichtung und mit einer tunnelförmigen Öffnung zur Aufnahme des Patienten oder Untersuchungsobjektes vorgesehen.

Derartige Diagnoseeinrichtungen werden zunehmend nicht nur im Bereich von Vor- oder Nachuntersuchungen eingesetzt, sondern werden vielfach auch unmittelbar in Operationssälen verwandt.

In diesen Fällen ist eine sterile Abdeckung notwendig, die verhindert, dass Patient und Gerät sich gegenseitig kontaminieren, da sich die Geräte selbst nicht oder nur schwierig sterilisieren lassen.

Im Stand der Technik ist hierzu beispielsweise aus der JP 07148147 A ein Computertomograph bekannt, bei dem Klettverschlüsse an der tunnelförmigen Öffnung vorgesehen sind und an einer sackartigen Hülle ebenfalls Klettverbindungen angebracht sind, so dass diese Hülle in der tunnelförmigen Öffnung angeordnet werden kann. Die sackförmige Hülle weist eine Öffnung auf, so dass ein Patient in die Hülle eingebracht werden kann. Diese Öffnung wird über einen Klettverschluss verschlossen. Über weitere in der sackförmigen Hülle vorgesehen Öffnungen kann Luft in die sackförmige Hülle zur Beatmung des Patienten eingebracht werden. Darüber hinaus sind in der Hülle Handschuhe angebracht, über die ein Arzt von außen in die Hülle hineingreifen und eine Operation beispielsweise am Kopf eines Patienten durchführen kann.

Nachteilig ist es hierbei, dass bereits vor der Operation entschieden werden muss, dass ein Patient in einer derartigen sackartigen Hülle unterzubringen ist. Darüber hinaus sind stets aufwendige Mittel zum Beatmen des Patienten in der Hülle vorzusehen. Schließlich besteht für die Ärzte während der Operation keine direkte Zugriffsmöglichkeit auf den Patienten, so dass im Falle von Komplikationen die Hülle geöffnet und der Patient vom Fußende her aus der Hülle herausgezogen werden oder die Hülle aufgeschnitten werden muss. Gerade bei Operationen am geöffneten Schädel stellt dies jedoch ein Problem dar.

Eine weitere Abdeckung ist darüber hinaus aus der DE 196 39 975 C1 bekannt, die eine medizinische Einrichtung mit einer tunnelförmigen Öffnung zur Aufnahme eines Untersuchungsobjektes beschreibt, wobei an der tunnelförmigen Öffnung eine Barriere vorgesehen ist, die die tunnelförmige Öffnung von der einen zur anderen Seite hin derart verschließt, dass das Untersuchungsobjekt sowohl von der einen als auch von der anderen Seite in die tunnelförmige Öffnung einbringbar ist. Insbesondere ist an der tunnelförmigen Öffnung eine sackartige Hülle vorgesehen, die entweder von der einen oder von der anderen Richtung durch die tunnelförmige Öffnung hindurchgestülpt werden kann, so dass Patienten entweder von der einen oder von der anderen Richtung in den Untersuchungsbereich einbringbar sind. Auf diese Weise sollen diese infektionsgefährdeten Patienten mit medizinischen Diagnose- oder Therapieeinrichtungen untersucht werden können, die auch für nicht infektionsgefährdete Patienten Anwendung finden.

Problematisch erscheint es jedoch hierbei, dass die Hülle nicht austauschbar ist und eine Sterilisation derselben nur schwierig durchgeführt werden kann. Ein Einsatz einer derartigen Einrichtung innerhalb eines Operationssaales erscheint daher nicht zweckmäßig.

Insbesondere bei der Operation von Patienten ist es notwendig, dass im Rahmen der Operation der Patient kurzzeitig zur weiteren Diagnose in das Diagnosegerät geschoben werden kann und dann die Operation hernach fortgesetzt wird, ohne dass die sterile Umgebung verlassen wird.

Es ist daher Aufgabe der Erfindung, eine Abdeckung für eine medizinische Einrichtung bereitzustellen, mit der verhindert wird, dass sich Patient und Gerät gegenseitig kontaminieren, wobei der Schutz des Patienten im Vordergrund steht und darüber hinaus eine gute Zugänglichkeit und Praktikabilität während einer Operation gegeben ist.

Die Erfindung löst diese Aufgabe durch Bereitstellung einer Abdeckung für eine tunnelförmige medizinische Einrichtung, insbesondere einen Computer- oder Kernspintomographen oder ein Strahlendiagnosegerät, bestehend aus einem beidseitig offenen Schlauch zum Anordnen in der tunnelförmigen Einrichtung, der im Bereich seiner beiden Enden Mittel zum Befestigen an der tunnelförmigen medizinischen Einrichtung aufweist und wobei ein Untersuchungsobjekt in den Schlauch einschiebbar ist.

Auf diese Weise kann besonders einfach eine Abdeckung des beispielsweise Computertomographen erfolgen. Die Öffnung bzw. der Tunnel im Diagnosegerät ist dabei innen allseitig umschlossen. Die Abdeckung wird in die tunnelförmige Öffnung eingeführt und die beiden Enden des beidseitig offenen Schlauches an der tunnelförmigen medizinischen Einrichtung, insbesondere auf beiden Seiten des Tunnels befestigt. Durch die Befestigung kann insbesondere auch vorgesehen sein, dass der offene Schlauch aufgespannt wird, so dass sich in der Diagnoseeinrichtung eine Art Röhre durch die Abdeckung bildet, in die ein Patient, der während einer Operation im Diagnosegerät untersucht werden soll, schnell und unproblematisch hineingeschoben werden kann. Nach der Operation können dann die beiden Enden, die die Mittel zum Befestigen aufweisen, wieder vom Diagnosegerät gelöst und die gesamte Abdeckung entsorgt werden.

Es kann dabei insbesondere vorgesehen sein, dass die Abdeckung einen zylindrischen Mittelteil aufweist, der im wesentlichen der Länge sowie dem Durchmesser der tunnelförmigen medizinischen Einrichtung entspricht und wobei auf jeder Seite des zylindrischen Mittelteils sich nach außen erweiternde Abschnitte vorgesehen sind. Durch die sich nach außen erweiternden Abschnitte werden trichterförmige Öffnungen gebildet, in die ein Patient gegebenenfalls samt OP-Tisch gut eingeschoben werden kann. Auch hier kann vorgesehen sein, dass die sich erweiternden Abschnitte im befestigten Zustand aufgespannt sind.

Es kann des weiteren vorgesehen sein, dass die Abdeckung aus verschiedenen Ringsegmenten zur Bildung der zuvor beschriebenen Form zusammengesetzt ist.

Insbesondere kann die Abdeckung aus einer Folie, einem textilen Material, einem Non-woven sowie einem mehrschichtigen Material, das eines oder mehrere der vorstehenden Materialien umfasst, bestehen. Das Abdeckungsmaterial kann dabei insbesondere beschichtet sein, beispielsweise um eine Flüssigkeitsdichtheit zu gewährleisten. Bevorzugt ist hierbei eine Herstellung der Abdeckung aus Polyethylen (PE), wobei das Material sterilisierbar und ausreichend transparent sein sollte, um eine genügende Durchlässigkeit für die zur Diagnose verwendete Strahlung zu besitzen.

Besonders vorteilhaft ist es, wenn als Mittel zum Befestigen die beiden Endbereiche der Abdeckung zumindest abschnittsweise elastifiziert sind und die elastifizierten Enden entsprechende Vorsprünge an der medizinischen Einrichtung im angebrachten Zustand hintergreifen. Als elastische Mittel können dabei in bzw. an den Endbereichen der Abdeckung elastische Bänder vorgesehen sein, die im vorgedehnten Zustand an der Abdeckung befestigt werden und dann nach der Befestigung kontrahieren und dadurch die Abdeckung offenhalten und an ihren Enden am Diagnosegerät fixieren.

Des weiteren betrifft die Erfindung eine medizinische Einrichtung, insbesondere ein Röntgen-, Computertomographie- oder Kernspintomographiegerät, umfassend eine tunnelförmige Diagnoseeinheit zur Aufnahme eines Patienten gegebenenfalls zusammen mit einem OP-Tisch und eine entfernbare, in der tunnelförmigen Diagnoseeinheit anordenbare schlauchartige Abdeckung, insbesondere mit den vorstehend beschriebenen Merkmalen, wobei die Abdeckung die tunnelförmige Diagnoseeinheit durchgreift und im Bereich ihrer beiden offenen Enden Mittel zum Befestigen an der Diagnoseeinheit vorgesehen sind.

Dabei kann die tunnelförmige Diagnoseeinheit an ihren beiden offenen Seiten einen im wesentlichen ringförmigen, die Öffnung umschließenden Vorsprung aufweisen, wobei die Abdeckung den Vorsprung hintergreift. Auf diese Weise kann besonders einfach eine Befestigung und ein Aufspannen der Abdeckung erzielt werden.

Insbesondere kann die Abdeckung an ihren Öffnungen elastische Mittel aufweisen, die im vorgedehnten Zustand zumindest abschnittsweise an der Abdeckung angebracht sind, wobei die Öffnungen eine solche Abmessung aufweisen, dass sie über den Vorsprung stülpbar sind und im gestülpten Zustand durch die elastischen Mittel hinter dem Vorsprung, den sie hintergreifen, festlegbar sind.

Auf diese Weise kann die Abdeckung durch einfaches Überstreifen unter Dehnung der elastischen Mittel an den Vorsprüngen, die an der Diagnoseeinheit angeordnet sind, festgelegt werden. Die Abdeckung kann dabei zumindest in dem Teil, der die tunnelförmige Diagnoseeinheit durchgreift, Abmessungen aufweisen, die in Länge und Durchmesser in etwa den Abmessungen der Diagnoseeinheit entsprechen. Auf diese Weise wird sichergestellt, dass für die Aufnahme des Patienten der gesamte Durchmesser und die gesamte Länge der Diagnoseeinheit zur Verfügung steht und die Abdeckung nicht unnötige Falten schlägt oder Stellen aufweist, an denen sie sich spannt.

Weitere Merkmale und Vorteile der Erfindung ergeben sich aus den übrigen Anmeldungsunterlagen. Alle Merkmale sind für die Erfindung einzeln oder in Kombination miteinander erfindungswesentlich.

Die Erfindung soll im Folgenden anhand einer Zeichnung näher erläutert werden. Dabei zeigen:
- Figur 1: eine medizinische Einrichtung und
- Figur 2: eine Abdeckung.

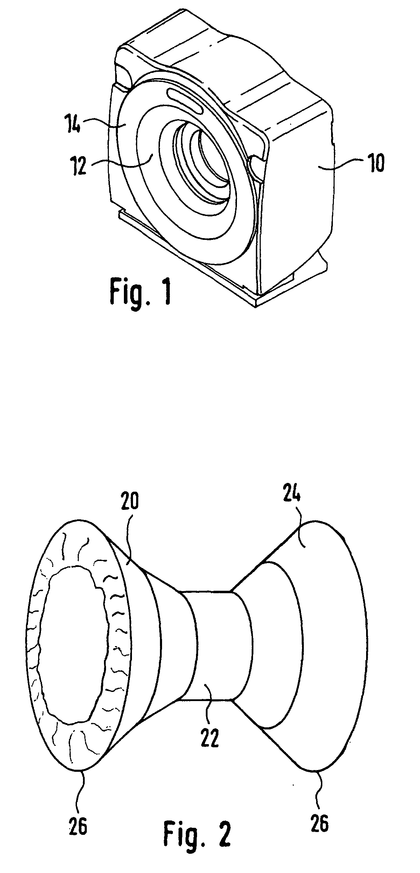

Figur 1 zeigt eine medizinische Einrichtung, hier einen Computertomographen, der in seiner Gesamtheit mit dem Bezugszeichen 10 versehen ist. Dieser umfasst eine tunnelförmige Diagnoseeinheit 12 zum Einschieben eines Untersuchungsobjektes, insbesondere eines Patienten. Die tunnelförmige Diagnoseeinrichtung 12 weist an beiden Enden des Tunnels einen im wesentlichen ringförmigen, die Öffnung umschließenden Vorsprung 14 auf.

Figur 2 zeigt eine erfindungsgemäße Abdeckung im - zur besseren Erkennbarkeit - gestreckten und auseinandergezogenen Zustand in axialer und radialer Richtung, die aus mehreren ringförmigen Abschnitten eines transparenten PE-Materials besteht. Die Abdeckung 20 besitzt dabei in der Mitte einen zylindrischen Abschnitt 22, der im wesentlichen hinsichtlich Länge und Durchmesser der Öffnung des Computertomographen 10 entspricht. Dieser zylindrische Mittelteil 22 durchgreift dabei den tunnelförmigen Abschnitt der Diagnoseeinheit 12. An den beiden Seiten des zylindrischen Mittelteils 22 sind sich nach außen erweiternde Seitenabschnitte 24 vorgesehen. Die Seitenabschnitte 24 weisen hierbei im Bereich ihrer Enden Elastifizierungsmittel in Form von an den Seitenabschnitten 24 angebrachten Gummibändern 26 auf, wobei die Seitenabschnitte 24 durch Dehnung der vorgedehnt aufgebrachten Gummibänder 26 um die Vorsprünge 14 herumlegbar bzw. stülpbar sind, so dass sich die Gummibänder 26 nach Herumlegen um den Vorsprung 16 wieder kontrahieren und so die Abdeckung 20 am Computertomographen 10 festlegen und die Abdeckung 20 darüber hinaus aufspannen. D. h. die Befestigungsmittel sind gleichzeitig die Aufspannmittel.

Des weiteren weist die Abdeckung Bereiche 28 auf. Die Bereiche 28, die außerhalb der Elastifizierungsmittel liegen, entstehen, da die Gummibänder 26 in eine Einschlagfalte der Abschnitte 24 eingelegt werden. Die Bereiche 28 besitzen darüber hinaus den Vorteil, dass die Elastifizierungsmittel um die Vorsprünge 16 herumgelegt werden können, ohne dass die anbringende Person mit der Innenseite der Abdeckung 20 in Kontakt kommt.

Soll nun während einer Operation eine Computertomographie durchgeführt werden, so kann der Patient entweder mit dem OP-Tisch oder auf einen gesonderten Tisch umgebettet werden und unmittelbar im Operationssaal in den zuvor mit der Abdeckung versehenen Computertomographen geschoben werden, woraufhin die entsprechenden Bilder angefertigt werden können. Die Operation kann dann fortgesetzt werden und der Computertomograph kann nach Beendigung der Operation durch Vorsehen einer neuen Abdeckung für den nächsten Patienten vorbereitet werden. Die Abdeckung wird dabei in der Regel vor Anbringung zusätzlich sterilisiert sein.

## Patentansprüche

1. Abdeckung für eine tunnelförmige medizinische Einrichtung (10), insbesondere einen Computer- oder Kernspintomographen, die in der tunnelförmigen Einrichtung anordenbar ist und die im Bereich seiner beiden Enden Mittel zum Befestigen (26) an beiden Seiten der tunnelförmigen medizinischen Einrichtung (10) aufweist, wobei ein Untersuchungsobjekt in den Schlauch einschiebbar ist, **dadurch gekennzeichnet dass** die Abdeckung aus einem beidseitig offenenen Schlauch besteht.

2. Abdeckung nach Anspruch 1, wobei die Abdeckung (20) einen zylindrischen Mittelteil (22) zum Durchgreifen der tunnelförmigen Einrichtung aufweist, der im wesentlichen der Länge sowie dem Durchmesser der tunnelförmigen medizinischen Einrichtung (10) entspricht.

3. Abdeckung nach Anspruch 1 oder 2, wobei die Mittel zum Befestigen (26) gleichzeitig als Aufspannmittel der Abdeckung (20) dienen.

4. Abdeckung nach einem der Ansprüche 1 bis 3, wobei auf jeder Seite des zylindrischen Mittelteils (22) sich nach außen erweiternde Abschnitte (24) vorgesehen sind.

5. Abdeckung nach einem der Ansprüche 1 bis 4, wobei die Abdeckung (20) aus einer Folie, einem textilen Material, einem Non-woven und/oder einem Laminat umfassend eines oder mehrere der vorstehenden Materialien besteht.

6. Abdeckung nach einem der Ansprüche 1 bis 5, wobei als Mittel zum Befestigen (26) die beiden Endbereiche der Abdeckung (20) zumindest abschnittsweise elastifiziert sind und die elastifizierten Endbereiche entsprechende Vorsprünge (14) an der medizinischen Einrichtung (10) im angebrachten Zustand hintergreifen.

7. Abdeckung nach Anspruch 6, wobei an den beiden Enden elastische Mittel, insbesondere elastische Bänder (26), vorgesehen sind, die im vorgedehnten Zustand an den Endbereichen (24) der Abdeckung (20) angebracht sind und dadurch die Endbereiche (24) der Abdeckung (20) elastifizieren.

8. Abdeckung nach einem der vorangehenden Ansprüche, wobei die Abdeckung (20) aus einem sterilisierbaren Material besteht.

9. Abdeckung nach einem der vorangehenden Ansprüche, wobei die Abdeckung (20) aus einem transparenten Material besteht.

10. Abdeckung nach einem der vorangehenden Ansprüche, wobei die Abdeckung (20) aus Polyethylen besteht.

11. Medizinische Einrichtung insbesondere Röntgen-, Computertomographie- oder Kernspintomographiegerät, umfassend eine tunnelförmige Diagnoseeinheit (12) zur Aufnahme eines Patienten und eine entfernbare in der tunnelförmigen Diagnoseeinheit (12) anordenbare Abdeckung (20), wobei die Abdeckung (20) die tunnelförmige Diagnoseeinheit (12) durchgreift und an ihren beiden Endbereichen Mittel zum Befestigen (20) an der Diagnoseeinheit (12) vorgesehen sind, **dadurch gekennzeichnet dass** die Abdeckung schlauchartig ist und die beiden offenen Endbereichen offen sind.

12. Medizinische Einrichtung nach Anspruch 11, wobei die tunnelförmige Diagnoseeinrichtung (12) an ihren beiden offenen Seiten einen im wesentlichen ringförmigen, die Öffnung umschließenden Vorsprung (14) aufweist, und die Abdeckung (20) den Vorsprung (14) lösbar hintergreift.

13. Medizinische Einrichtung nach Anspruch 12, wobei die Öffnungen der Abdeckung (20) elastische Mittel (26) aufweisen, die im vorgedehnten Zustand an der Abdeckung (20) angebracht sind, wobei die Öffnungen eine solche Abmessung aufweisen, dass sie über den Vorsprung (14) stülpbar sind und im gestülpten Zustand durch die elastischen Mittel (26) festgelegt sind.

14. Medizinische Einrichtung nach einem der Ansprüche 11 bis 13, wobei zumindest ein Teil der Abdeckung (20), der die tunnelförmige Diagnoseeinheit (12) durchgreift, Abmessungen aufweist, die in Länge und Durchmesser in etwa den Abmessungen der Diagnoseeinheit (12) entsprechen.

## Claims

1. Enclosure for a tunnel-shaped medical apparatus (10), in particular a computer- or nuclear spin tomograph, which can be arranged in the tunnel-shaped apparatus and in the vicinity of both its ends has means for fastening (26) on both sides of the tunnel-shaped medical apparatus (10), whereby an object for investigation can be pushed into the tube, **characterised in that** the enclosure consists of a tube open on either side.

2. Enclosure according to Claim 1, whereby the enclosure (20) has a cylindrical centre section (22) extending through the tunnel-shaped apparatus, which substantially corresponds to the length as well as the diameter of the tunnel-shaped medical apparatus (10).

3. Enclosure according to Claim 1 or 2, whereby the means for fastening (26) at the same time serve as means for stretching the enclosure (20).

4. Enclosure according to any one of Claims 1 to 3, whereby sections (24) extending outward are provided on either side of the cylindrical centre section (22).

5. Enclosure according to any one of Claims 1 to 4, whereby the enclosure (20) consists of a foil, textile material, non-woven and/or laminate comprising one or several of the above materials.

6. Enclosure according to any one of Claims 1 to 5, whereby as means for fastening (26) the two end regions of the enclosure (20) are elasticized at least in sections and the elasticized end regions engage behind corresponding projections (14) on the medical apparatus (10) in the attached condition.

7. Enclosure according to Claim 6, whereby elastic means are provided at the two ends, in particular elastic hoops (26), which are attached in the pre-stretched condition to the end regions (24) of the enclosure (20) and as a result elasticize the end regions (24) of the enclosure (20).

8. Enclosure according to any one of the preceding claims, whereby the enclosure (20) consists of a sterilizable material.

9. Enclosure according to any one of the preceding Claims, whereby the enclosure (20) consists of a transparent material.

10. Enclosure according to any one of the preceding Claims, whereby the enclosure (20) consists of polyethylene.

11. Medical apparatus in particular X-ray, computer tomography or nuclear spin tomography equipment, comprising a tunnel-shaped diagnostic unit (12) for admitting a patient and a removable enclosure (20), which can be arranged in the tunnel-shaped diagnostic unit (12), whereby the enclosure (20) extends through the tunnel-shaped diagnostic unit (12) and means for fastening (20) to the diagnostic unit (12) are provided at its two end regions, **characterised in that** the enclosure is tubular and the two open end regions are open.

12. Medical apparatus according to Claim 11, whereby the tunnel-shaped diagnostic apparatus (12) at its two open sides has a substantially circular projection (14) surrounding the opening and the enclosure (20) detachably engages behind the projection (14).

13. Medical apparatus according to Claim 12, whereby the openings of the enclosure (20) have elastic means (26), which are attached in the pre-stretched condition to the enclosure (20), whereby the openings have such a dimension that they can be clinched over the projection (14) and are fixed in the clinched condition by the elastic means (26).

14. Medical apparatus according to any one of Claims 11 to 13, whereby at least part of the enclosure (20), which extends through the tunnel-shaped diagnostic unit (12), has dimensions, which in length and diameter roughly correspond to the dimensions of the diagnostic unit (12).

## Revendications

1. Recouvrement pour un dispositif médical en forme de tunnel (10), en particulier un tomographe à calculateur intégré ou à résonance magnétique nucléaire, qui peut être agencé dans le dispositif en forme de tunnel et présente, dans la zone de ses deux extrémités, des moyens pour la fixation (26) sur les deux côtés du dispositif médical en forme de tunnel (10), un objet d'exploration pouvant être introduit dans le tuyau,
**caractérisé en ce que** le recouvrement est constitué d'un tuyau ouvert des deux côtés.

2. Recouvrement selon la revendication 1, le recouvrement (20) présentant une partie centrale cylindrique (22) pour pénétrer dans le dispositif en forme de tunnel, laquelle correspond essentiellement à la longueur ainsi qu'au diamètre du dispositif médical en forme de tunnel (10).

3. Recouvrement selon la revendication 1 ou 2, les moyens pour la fixation (26) servant en même temps de moyens de serrage du recouvrement (20).

4. Recouvrement selon l'une des revendications 1 à 3, des sections (24) s'élargissant vers l'extérieur étant prévues de chaque côté de la partie centrale cylindrique (22).

5. Recouvrement selon l'une des revendications 1 à 4, le recouvrement (20) étant constitué d'un film, d'une matière textile, d'un non-tissé et/ou d'un aggloméré stratifié comportant un ou plusieurs des matériaux précédents.

6. Recouvrement selon l'une des revendications 1 à 5, les deux zones d'extrémité du recouvrement (20), en tant que moyens pour la fixation (26), étant élastifiées au moins par endroits et les zones d'extrémité élastifiées passant, dans l'état installé, derrière des saillies correspondantes (14) situées sur le dispositif médical (10).

7. Recouvrement selon la revendication 6, des moyens élastiques, notamment des bandes élastiques (26), étant prévus aux deux extrémités, lesquels sont amenés dans l'état pré-étiré sur les zones d'extrémité (24) du recouvrement (20) et élastifient ainsi les zones d'extrémité (24) du recouvrement (20).

8. Recouvrement selon l'une des revendications précédentes, le recouvrement (20) étant réalisé dans un matériau stérilisable.

9. Recouvrement selon l'une des revendications précédentes, le recouvrement (20) étant réalisé dans un matériau transparent.

10. Recouvrement selon l'une des revendications précédentes, le recouvrement (20) étant réalisé en polyéthylène.

11. Dispositif médical, en particulier un appareil de tomographie à résonance magnétique nucléaire, à calculateur intégré ou à rayons X, comportant une unité de diagnostic en forme de tunnel (12) pour recevoir un patient et un recouvrement (20) pouvant être agencé dans l'unité de diagnostic en forme de tunnel (12) et enlevé, le recouvrement (20) pénétrant dans l'unité de diagnostic en forme de tunnel (12) et des moyens pour la fixation (26) sur l'unité de diagnostic (12) étant prévus sur ses deux zones d'extrémité,
**caractérisé en ce que** le recouvrement est en forme de tuyau et que les deux zones d'extrémité sont ouvertes.

12. Dispositif médical selon la revendication 11, le dispositif de diagnostic en forme de tunnel (12) présentant sur ses deux côtés ouverts une saillie (14) essentiellement circulaire entourant l'ouverture, et le recouvrement (20) passant derrière la saillie (14) de manière amovible.

13. Dispositif médical selon la revendication 12, les ouvertures du recouvrement (20) présentant des moyens élastiques (26) qui sont amenés dans l'état pré-étiré sur le recouvrement (20), les ouvertures présentant une dimension telle qu'elles peuvent être retournées sur les saillies (14) et être fixées dans l'état retourné par les moyens élastiques (26).

14. Dispositif médical selon l'une des revendications 11 à 13, une partie au moins du recouvrement (20) qui pénètre dans l'unité de diagnostic en forme de tunnel (12) présentant des dimensions qui correspondent en longueur et diamètre approximativement aux dimensions de l'unité de diagnostic (12).
